# EUROPEAN PATENT APPLICATION

(11) **EP 2 209 303 A2**
(43) Date of publication of application: **21.07.2010**
(21) Application number: 10150635.0
(22) Date of filing: 13.01.2010
(51) Int. Cl.: H04N 5/232, A61B 1/04, G06T 5/00

(54) **Image processing system, image processing method, and computer readable medium**

(30) Priority: 14.01.2009 JP 2009005839
(71) Applicant: FUJIFILM Corporation, Tokyo 106-8620 (JP)
(72) Inventor: Yamaguchi, Hiroshi, Kanagawa (JP); Saitou, Takaaki, Kanagawa (JP); Maeda, Kiyohiro, Kanagawa (JP)
(74) Representative: Klunker . Schmitt-Nilson . Hirsch

(57) **Abstract**

Provided are an image processing system, an image processing method, and a program for adjusting an image quality of a fluorescent light motion image. An image obtaining section that obtains a high image-quality motion image and a low image-quality motion image in which a same subject is captured simultaneously; a motion calculating section that calculates a motion of the subject in the high image-quality motion image, from the high image-quality motion image; and an image adjusting section that generates a motion image resulting from adjusting an image quality of the low image-quality motion image, based on the motion calculated by the motion calculating section are included, to adjust an image quality of a low image-quality motion image. Accordingly, the image quality of a low image-quality image can be improved.

## Description

### TECHNICAL FIELD

The present invention relates to an image processing system, an image processing method, and a computer readable medium for adjusting the image quality of a low image-quality motion image.

### BACKGROUND ART

Japanese Patent Application Publication No. H07-250804 (Patent Document No. 1) discloses a technique of detecting the motion vector of a fluorescent light image, to perform motion compensation to the fluorescent light image.

### DISCLOSURE OF THE INVENTION

### PROBLEMS TO BE SOLVED BY THE INVENTION

Patent Document No. 1 uses a fluorescent light motion image to detect the motion vector. However, a fluorescent light image generally has a long exposure time and a low frame rate, which would generate a large amount of blurring and prevent detection of accurate motion vectors. As a result, the technology cannot improve the image quality of the fluorescent light motion image to a satisfactory level.

### MEANS FOR SOLVING THE PROBLEMS

According to a first aspect of the innovations herein, provided is an image processing system including: an image obtaining section that obtains a high image-quality motion image and a low image-quality motion image in which a same subject is captured simultaneously; a motion calculating section that calculates a motion of the subject in the high image-quality motion image, from the high image-quality motion image; and an image adjusting section that generates a motion image resulting from adjusting an image quality of the low image-quality motion image, based on the motion calculated by the motion calculating section.

The image obtaining section may obtain the low image-quality motion image generated by receiving fluorescent light from the subject irradiated with excitation light, and the high image-quality motion image generated by receiving reflected light from the subject.

The image processing system includes: an irradiating section that sequentially irradiates the subject with the excitation light and visible light, by switching between the excitation light and the visible light; and an image capturing section that sequentially captures images of fluorescent light from the subject irradiated with the excitation light and images of reflected light from the subject irradiated with the visible light, where the image obtaining section obtains a motion image of the fluorescent light and a motion image of the reflected light that have been captured by the image capturing section.

The image processing system includes: an irradiating section that irradiates the subject with the excitation light; a first image capturing section that captures images of fluorescent light from the subject irradiated with the excitation light; and a second image capturing section that captures images of reflected light from the subject irradiated with the excitation light, where the image obtaining section obtains a motion image of the fluorescent light captured by the first image capturing section and a motion image of the reflected light captured by the second image capturing section.

The image obtaining section may obtain the high image-quality motion image whose exposure time is shorter than an exposure time in which the low image-quality motion image is exposed, and the image adjusting section may generate motion image constituting images by correcting blurring in motion image constituting images included in the low image-quality motion image, based on the motion calculated by the motion calculating section.

The image obtaining section may obtain the high image-quality motion image whose frame rate is higher than a frame rate of the low image-quality motion image, and the image adjusting section may generate a interpolation image for interpolating motion image constituting images included in the low image-quality motion image, based on the motion calculated by the motion calculating section.

The image obtaining section may obtain the high image-quality motion image whose resolution is higher than a resolution of the low image-quality motion image, and the image adjusting section may render, in a high resolution, motion image constituting images included in the low image-quality motion image, using the motion calculated by the motion calculating section.

According to a second aspect of the innovations herein, provided is an image processing method for processing an image by means of a computer, including: obtaining a high image-quality motion image and a low image-quality motion image in which a same subject is captured simultaneously; calculating a motion of the subject in the high image-quality motion image, from the high image-quality motion image; and adjusting an image quality of the low image-quality motion image, using the calculated motion.

According to a third aspect of the innovations herein, provided is a computer readable medium for storing a program, the program causing a computer to function as: an image obtaining section that obtains a high image-quality motion image and a low image-quality motion image in which a same subject is captured simultaneously; a motion calculating section that calculates a motion of the subject in the high image-quality motion image, from the high image-quality motion image; and an image adjusting section that adjusts an image quality of the low image-quality motion image, using the motion calculated by the motion calculating section.

The summary of the invention does not necessarily describe all necessary features of the present invention. The present invention may also be a subcombination of the features described above.

### BRIEF DESCRIPTION OF THE DRAWINGS

Fig. 1 shows an example of an image processing system 100 of an embodiment.
Fig. 2 shows an example of an image processing section 102.
Fig. 3 shows an example of a rotation filter 108 of an irradiating section 105.
Fig. 4 shows an example of correspondence between a light source 107 and the rotation filter 108.
Fig. 5 shows an example of an image capturing section 112.
Fig. 6 shows an example of a rotation filter 151.
Fig. 7 shows an example of a time chart of an exposure time for an image capturing section 112.
Fig. 8 shows another example of the image capturing section 112.
Fig. 9A and Fig. 9B show an example of a time chart of an exposure time for a first image capturing section 171 and a second image capturing section 172.

### BEST MODE FOR CARRYING OUT THE INVENTION

The invention will now be described based on the preferred embodiments, which do not intend to limit the scope of the present invention, but exemplify the invention. All of the features and the combinations thereof described in the embodiment are not necessarily essential to the invention.

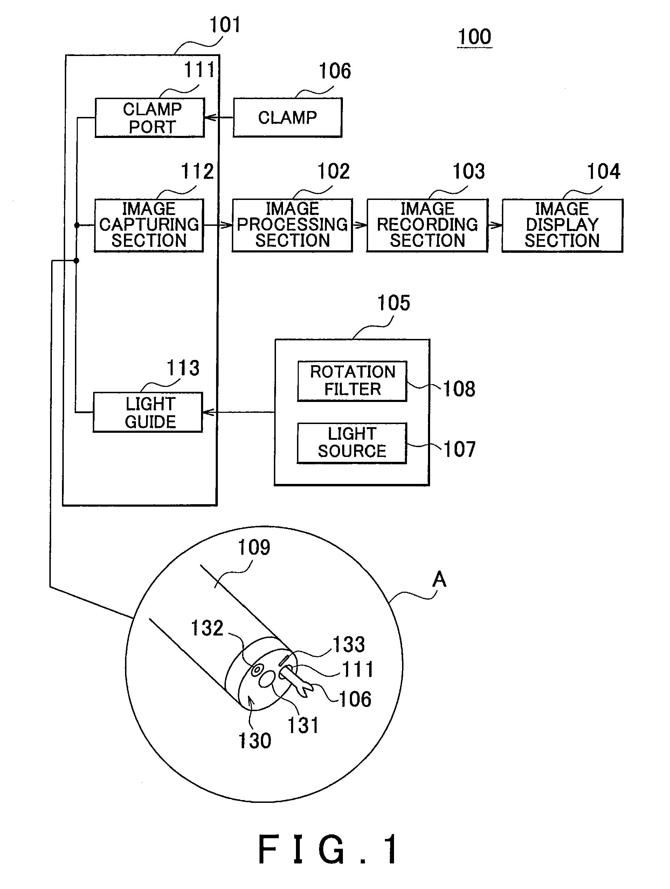

Fig. 1 shows an example of an image processing system 100 of an embodiment. The present embodiment explains the image processing system as an endoscope system. The image processing system 100 includes an endoscope 101, an image processing section 102, an image recording section 103, an image display section 104, an irradiating section 105, and a clamp 106. Note that the section A in Fig. 1 is an enlarged view of a tip 109 of the endoscope 101.

The endoscope 101 includes a clamp port 111, an image capturing section 112, and a light guide 113. The tip 109 of the endoscope 101 has, on its end surface 130, a lens 131 as a part of the image capturing section 112. The tip 109 also has an outlet 132 as a part of the light guide 113 at its end surface 130. A clamp 106 is inserted to the clamp port 111. The clamp port 111 guides the clamp 106 towards the tip 109. Note that the form of the tip of the clamp 106 may be varied. Moreover, a various type of devices, other than the clamp 106, may be inserted to the clamp port 111, for treating a living body. A nozzle 133 delivers water or air to outside.

The irradiating section 105 irradiates a subject with light. The irradiating section 105 irradiates a subject with excitation light. The irradiating section 105 also irradiates a subject with white light. The white light is an example of visible light.
The irradiating section 105 may sequentially irradiate a subject with excitation light and white light by switching therebetween. The irradiating section 105 includes a light source 107 and a rotation filter 108. The light source 107 emits white light. The light source 107 may be an electric bulb or an LED. The rotation filter 108 includes a first filter transmitting white light and a second filter transmitting excitation light. The irradiating section 105 switches between the white light and the excitation light by rotating the rotation filter 108, thereby irradiating the subject with the white light and the excitation light. An arrangement is possible in which the tip 109 of the endoscope 101 be provided with the irradiating section 105. For example, the tip 109 of the endoscope 101 may be provided with an LED emitting the white light and an LED emitting the excitation light.

The light guide 113 is made up of optical fibers, for example. The light guide 113 guides light emitted from the irradiating section 105 towards the tip 109 of the endoscope 101. The light emitted from the irradiating section 105 is guided through the light guide 113 to be outputted from the outlet 132 on the end surface 130 to irradiate the subject.

The image capturing section 112 simultaneously captures a low image-quality motion image and a high image-quality motion image of a same subject. The image capturing section 112 captures returned light from a subject being an observation target irradiated by the irradiating section 105. The image capturing section 112 captures an image of fluorescent light from a subject irradiated with excitation light. In addition, the image capturing section 112 may capture an image of reflected light from a subject irradiated with white light. The image capturing section 112 may also capture an image of reflected light from a subject irradiated with excitation light. In addition, the image capturing section 112 may sequentially and alternately capture the image of the fluorescent light from the subject irradiated with the excitation light and the image of the reflected light from the subject. Since fluorescent light is weak, the image capturing section 112 may capture an image of fluorescent light in a longer exposure time than when capturing an image of reflected light. Moreover, the image capturing section 112 may capture a motion image of fluorescent light at a lower frame rate than a frame rate at which a motion image of reflected light is taken. Also because of weakness of fluorescent light, the image capturing section 112 may capture an image of fluorescent light with higher sensitivity than for an image of reflected light. When a motion image of fluorescent light is captured in a longer exposure time than in capturing a motion image of reflected light, the subject in the captured image will fluctuate much. When a motion image of fluorescent light is captured at a lower frame rate than for a motion image of reflected light, the motion of the subject in the captured image is not reproduced in detail. For having better sensitivity for the fluorescent light motion image than the reflected light motion image, it is necessary to add the accumulated pixel charge when reading it, or to enlarge the size of the pixel itself. Therefore, the fluorescent light motion image has lower resolution, to result in low image quality for the fluorescent light motion image and high image quality for the reflected light motion image.

The image processing section 102 obtains a motion image captured by the image capturing section 112. The image processing section 102 obtains a low image-quality motion image and a high image-quality motion image of a same subject that have been simultaneously captured by the image capturing section 112. The image processing section 102 obtains a fluorescent light motion image and a reflected light motion image captured by the image capturing section 112. The image processing section 102 may obtain a low image-quality motion image generated by receiving fluorescent light from a subject irradiated with excitation light, and a high image-quality motion image generated by receiving reflected light from the subject. In addition, the image processing section 102 performs image processing to a low image-quality motion image, based on the obtained high image-quality motion image. In addition, the image processing section 102 may be realized by an information processing apparatus such as a CPU, or by electronic circuitry or electric circuitry.

The image recording section 103 records a low image-quality motion image having undergone image processing in the image processing section 102. The image recording section 103 also records a high image-quality motion image obtained by the image processing section 102. The image recording section 103 may also record a low image-quality motion image obtained by the image processing section 102. The image recording section 103 may include a recording medium such as a flash memory, and a recording control section for recording an image to the recording medium. The recording control section may be realized by an information processing apparatus such as a CPU.

The image display section 104 displays a motion image recorded by the image recording section 103. The image display section 104 displays a recorded high image-quality motion image. The image display section 104 also displays a recorded low image-quality motion image. The image display section 104 may include a display such as a liquid crystal display, an organic EL display, and a plasma display, and a display control section controlling the display. The display control section may be realized by an information processing apparatus such as a CPU.

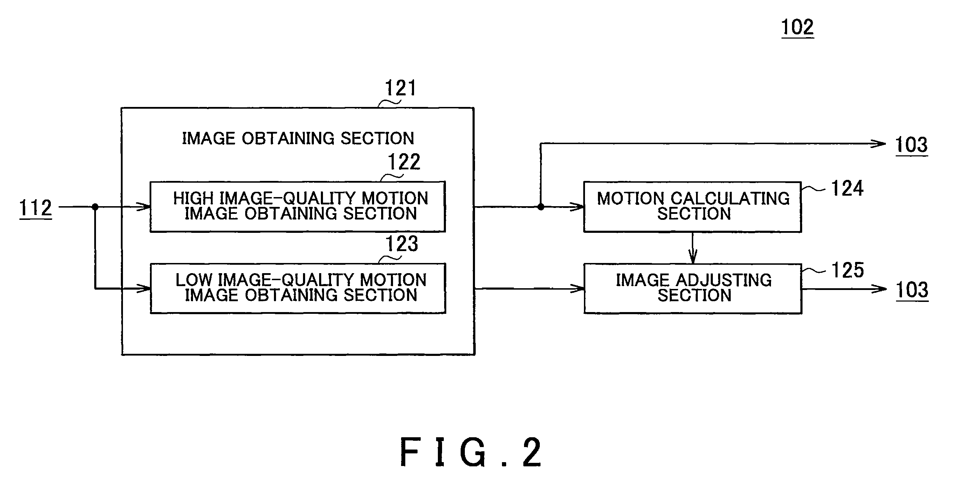

Fig. 2 shows an example of the image processing section 102. The image processing section 102 includes an image obtaining section 121, a motion calculating section 124, and an image adjusting section 125. The image obtaining section 121 includes a high image-quality motion image obtaining section 122 and a low image-quality motion image obtaining section 123. The high image-quality motion image obtaining section 122 obtains a high image-quality motion image captured by the image capturing section 112. The high image-quality motion image obtaining section 122 may obtain a motion image of reflected light captured by the image capturing section 112. The high image-quality motion image obtaining section 122 outputs the obtained high image-quality motion image to the motion calculating section 124. The high image-quality motion image obtaining section 122 may also output the obtained high image-quality motion image to the image recording section 103. The low image-quality motion image obtaining section 123 obtains a low image-quality motion image captured by the image capturing section 112. The low image-quality motion image obtaining section 123 may obtain a motion image of fluorescent light captured by the image capturing section 112. The low image-quality motion image obtaining section 123 outputs the obtained low image-quality motion image to the image adjusting section 125.

The motion calculating section 124 calculates the motion of the subject in the high image-quality motion image obtained by the high image-quality motion image obtaining section 122. That is, the motion calculating section 124 calculates the motion vector of the subject in the high image-quality motion image obtained by the high image-quality motion image obtaining section 122, by block matching and so on. The motion calculating section 124 outputs the calculated motion of the subject to the image adjusting section 125. The image adjusting section 125 adjusts the image quality of the low image-quality motion image obtained by the low image-quality motion image obtaining section 123, using the motion calculated by the motion calculating section 124.

When a high image-quality motion image is exposed in a shorter exposure time than a low image-quality motion image, the image adjusting section 125 may correct the blurring in the motion image constituting images making up the low image-quality motion image, using the calculated motion. Specifically, when the exposure time gets long, the image is more prone to cause blurring. Therefore, the blurring is corrected to either eliminate or reduce the blurring. The motion image constituting images are images making up a motion image, and include a frame image, a field image, etc. When the frame rate is higher for the high image-quality motion image than for the low image-quality motion image, the image adjusting section 125 may generate an interpolation image for interpolating the motion image constituting images included in the low image-quality motion image, using the calculated motion. That is, the frame rate of the low image-quality motion image is increased by generating the interpolation images for interpolating the motion image constituting images among the frames of the low image-quality motion image. In addition, when the high image-quality motion image has a resolution higher than the low image-quality motion image, the image adjusting section 125 may raise the resolution of the motion image constituting images in the low image-quality motion image, thereby raising the resolution of the low image-quality motion image. The image adjusting section 125 outputs the adjusted low image-quality motion image to the image recording section 103.

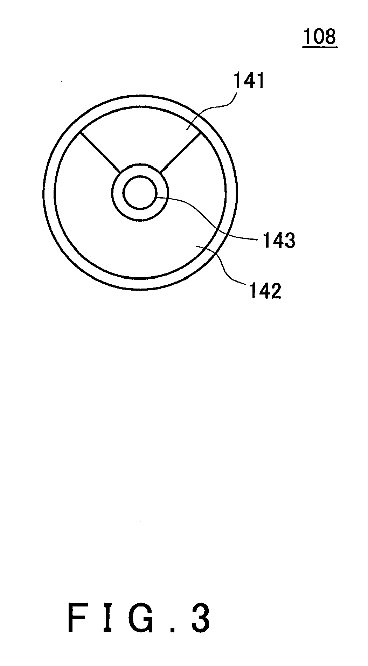

Fig. 3 shows an example of a rotation filter 108 of an irradiating section 105. The rotation filter 108 includes a first filter 141, a second filter 142, and a shaft 143. In the rotation filter 108, the first filter 141 and the second filter 142 are arranged along a circle. The shaft 143 being the center of rotation is provided in the center of the rotation filter 108. The first filter 141 transmits white light. The first filter 141 may transmit the light emitted from the light source 107 as it is. An aperture may be provided instead of the first filter 141. The second filter 142 transmits a wavelength band of the excitation light. The circumferential length of the first filter 141 is shorter than the circumferential length of the second filter 142.

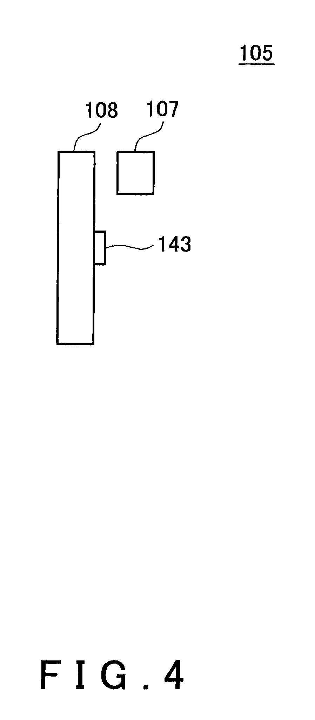

Fig. 4 shows an example of correspondence between a light source 107 and the rotation filter 108. The irradiating section 105 rotates the rotation filter 108 with the shaft 143 being a center of rotation, thereby alternately arranging the first filter 141 and the second filter 142 on the optical path of the light emitted from the light source 107. The irradiating section 105 rotates this rotation filter 108 to switch between the white light and the excitation light, to irradiate a subject. Since the circumferential length of the first filter 141 is shorter than the circumferential length of the second filter 142, the irradiating section 105 irradiates the excitation light in a longer irradiation period than for the white light. The irradiating section 105 includes a control section for controlling the light source 107 and the rotation filter 108. This control section may be realized by an information processing apparatus such as a CPU.

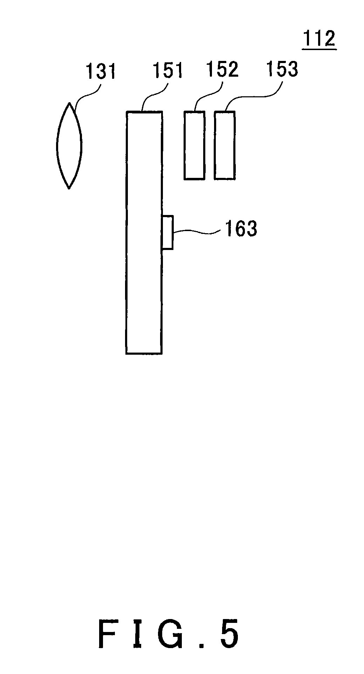

Fig. 5 shows an example of an image capturing section 112. The image capturing section 112 includes a lens 131, a rotation filter 151, a color filter 152, and an image capturing element 153. From the light transmitted through the lens 131, the image capturing element 153 receives light transmitted through the rotation filter 151 and the color filter 152. The color filter 152 may be an RGB color filter, or may be other color filters. The rotation filter 151 includes an aperture and an excitation light cutting filter for cutting the light of the wavelength band of excitation light and transmitting the light of the other wavelength bands. The image capturing section 112 rotates the rotation filter 151 with the shaft 163 being a center of rotation, to alternately arrange the excitation light cutting filter and the aperture on the optical path between the lens 131 and the image capturing element 153.

When the irradiating section 105 is emitting excitation light, the image capturing section 112 locates the excitation light cutting filter on the optical path. When the irradiating section 105 is emitting white light, the image capturing section 112 arranges the aperture on the optical path. By doing so, the image capturing element 153 is able to capture a fluorescent light image when the irradiating section 105 emits excitation light. That is, when the excitation light is emitted, reflected light of the excitation light and fluorescent light, which are from the subject, are incident to the image capturing section 112. However, since the excitation light cutting filter cuts off the excitation light, the image capturing element 153 can capture the fluorescent light image. When the irradiating section 105 is emitting white light, the image capturing element 153 is able to capture a color image. Note that the exposure time, the frame rate, etc. of the image capturing element 153 are controlled by an information processing apparatus such as a CPU. The rotation of the rotation filter 151 is controlled by an information processing apparatus. Note that the image capturing section 112 includes an image capturing element driver for driving the image capturing element 153. This image capturing element driver is controlled by an information processing apparatus such as a CPU. The information processing apparatus may be provided in the image capturing section 112, or in the image processing system 100.

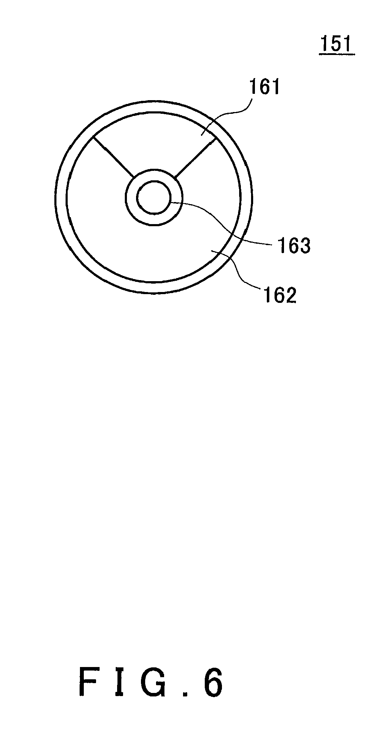

Fig. 6 shows an example of the rotation filter 151. The rotation filter 151 includes an aperture 161, an excitation light cutting filter 162, and a shaft 163. In the rotation filter 151, an aperture 161 and an excitation light cutting filter 162 are arranged along a circle. Instead of the aperture 161, the rotation filter 151 may be provided with a filter transmitting the incident light as it is. The circumferential length of the aperture 161 is shorter than the circumferential length of the excitation light cutting filter 162. Accordingly, the period in which the aperture 161 is on the optical path between the lens 131 and the image capturing element 153 is longer than the period in which the excitation light cutting filter 162 is on the optical path. In addition, within the period in which the first filter 141 of the rotation filter 108 is on the optical path of the light source 107, the aperture 161 of the rotation filter 151 is on the optical path between the lens 131 and the image capturing element 153. In addition, within the period in which the second filter 142 of the rotation filter 108 is on the optical path of the light source 107, the excitation light cutting filter 162 of the rotation filter 151 is on the optical path between the lens 131 and the image capturing element 153. That is, the irradiation start timing of the white light by the rotation filter 108 is substantially synchronized with the transmission start timing of the white light by the rotation filter 151. In addition, the irradiation start timing of the excitation light by the rotation filter 108 is substantially synchronized with the excitation light cutting start timing by the rotation filter 151.

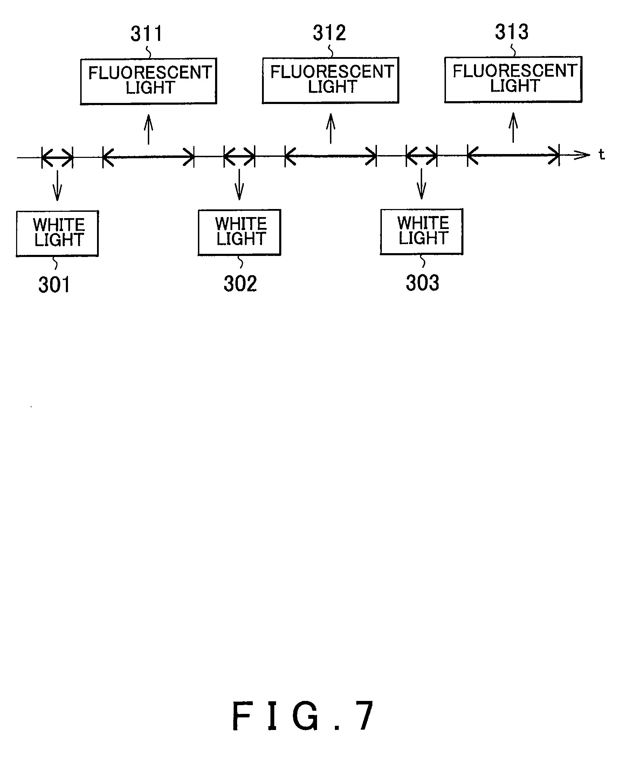

Fig. 7 shows an example of a time chart of an exposure time for an image capturing section 112. The lateral axis indicates a time. A short arrow in both directions indicates an exposure period for the reflected light of the white light. A long arrow in both directions indicates an exposure period for fluorescent light. The exposure time of the reflected light of the white light is shorter than the exposure time of the fluorescent light. Accordingly, the irradiating time of the white light is shorter than the irradiating time of the excitation light. The fluorescent light is weak, and so has an exposure time longer than an exposure time of reflected light. First, when the white light is emitted, the image capturing section 112 captures a white light image 301 by performing exposure using the reflected light of the white light. Next, when the excitation light is emitted, the image capturing section 112 captures a fluorescent light image 311 by performing exposure using the fluorescent light. After this, when the white light is emitted, the image capturing section 112 captures a white light image 302 by performing exposure using the reflected light of the white light. By performing the stated operation, the white light motion image and the fluorescent light motion image are alternately captured, in such an order as a white light image 301, a fluorescent light image 311, a white light image 302, a fluorescent light image 312, a white light image 303, a fluorescent light image 313, and so on. The white light image will be a color image. The white light images make up a reflected light motion image. The fluorescent light images make up a fluorescent light motion image.

The following explains the operation of the image processing system 100. The irradiating section 105 switches between the white light and the excitation light by rotating the rotation filter 108, thereby irradiating the subject with the white light and the excitation light. The image capturing section 112 rotates the rotation filter 151. Accordingly, the image capturing section 112 can concurrently capture the fluorescent light motion image and the reflected light motion image. Here, since fluorescent light is weak, the image capturing section 112 receives fluorescent light in an exposure time longer than an exposure time in which reflected light is received. Accordingly, the irradiating section 105 irradiates excitation light in a longer time than a time in which white light is irradiated. That is, the irradiating section 105 emits white light and fluorescent light by switching therebetween, in synchronization with the timings at which the image capturing section 112 starts receiving reflected light and fluorescent light.

To be specific, the image capturing section 112 rotates the rotation filter 151, to arrange the aperture 161 on the optical path between the lens 131 and the image capturing element 153 while the reflected light is captured. While the fluorescent light is captured, the excitation light cutting filter 162 is arranged on the optical path. In synchronization with this, the irradiating section 105 rotates the rotation filter 108, to arrange the first filter 141 on the optical path of the light source 107 while reflected light is captured, and to arrange the second filter on the optical path of the light source 107 while the fluorescent light is captured. As a result, by emitting white light, a color image of a subject can be captured, and by emitting excitation light, a fluorescent light can be captured. By alternately repeating the period for capturing a reflected light image and the period for capturing a fluorescent light image, the image capturing section 112 can alternately capture a reflected light motion image and a fluorescent light motion image, and can capture a color motion image and a fluorescent light motion image simultaneously. Here, the term "simultaneous(ly)" mean that the motion image constituting images making up a reflected light motion image and the motion image constituting images making up a fluorescent light motion image are captured simultaneously as a motion image, but does not mean that these motion image constituting images are captured at the same time of day.

Since fluorescent light is weak, the image capturing section 112 may read the pixel charge of the image capturing element 153 having received fluorescent light by adding the charge together. The image capturing section 112 may capture a reflected light motion image at a frame rate shorter than a frame rate for a fluorescent light motion image. In this case, the number of times at which the reflected light is captured is set larger than the number of times at which the fluorescent light is captured, instead of alternately capturing the reflected light and the fluorescent light.

The high image-quality motion image obtaining section 122 sequentially obtains color images captured by the image capturing section 112. As a result, the high image-quality motion image obtaining section 122 can obtain a reflected light motion image. The low image-quality motion image obtaining section 123 sequentially obtains fluorescent light images captured by the image capturing section 112. As a result, the low image-quality motion image obtaining section 123 can obtain a fluorescent light motion image captured by the image capturing section 112. The motion calculating section 124 calculates the motion of a subject from the reflected light motion image obtained by the high image-quality motion image obtaining section 122. The image adjusting section 125 adjusts the image quality of the fluorescent light motion image obtained by the low image-quality motion image obtaining section 123, using the motion calculated by the motion calculating section 124. The image adjusting section 125 adjusts the blurring in the motion image constituting images included in the fluorescent light motion image, using the motion calculated by the motion calculating section 124, when the exposure time is shorter for the reflected light motion image than for the fluorescent light motion image. That is, the motion can be obtained more accurately from an image having a shorter exposure time since it has a small amount of blurring, which helps adjust the image quality of the low image-quality motion image. When the frame rate is higher for the reflected light motion image than for the fluorescent light motion image, the image adjusting section 125 interpolates the motion image constituting images included in the fluorescent light motion image, using the motion calculated by the motion calculating section 124. That is, the motion of a subject is reproduced with more details for a motion image having a higher frame rate, and so the motion can be obtained more accurately from such a motion image, which helps adjust the image quality of the low image-quality motion image. When the resolution is higher for the reflected light motion image than for the fluorescent light motion image, the image adjusting section 125 may render the fluorescent light motion image in a higher resolution, using the motion calculated by the motion calculating section 124. In other words, the motion of a subject is reproduced with more details for a motion image having a higher resolution, and so the motion can be obtained more accurately from such a motion image, which helps adjust the image quality of the low image-quality motion image.

The image recording section 103 records the reflected light motion image obtained by the high image-quality motion image obtaining section 122 and the fluorescent light motion image adjusted by the image adjusting section 125. The image display section 104 displays the reflected light motion image and the fluorescent light motion image recorded by the image recording section 103. The image display section 104 may display the reflected light motion image and the fluorescent light motion image simultaneously. Specifically, the first display region may display the reflected light motion image, and the second display region may display the fluorescent light motion image. In addition, the image display section 104 may display one of the reflected light motion image and the fluorescent light motion image. For example, display of the reflected light motion image and the fluorescent light motion image can be switched in response to a switching instruction from a user.

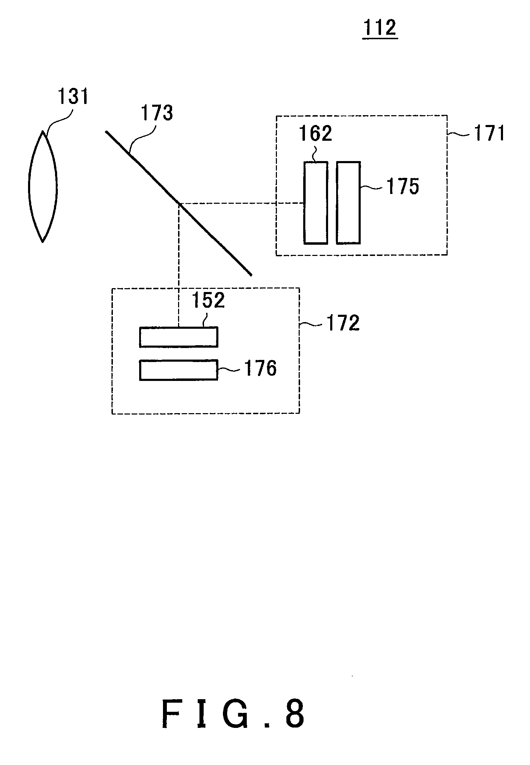

Fig. 8 shows another example of the image capturing section 112. The image capturing section 112 includes a lens 131, a first image capturing section 171, a second image capturing section 172, and a half mirror 173. The half mirror 173 transmits a part of light, and reflects the rest of the light, thereby splitting the light having been transmitted through the lens 131, into two rays of light. The half mirror 173 transmits the amount of light that is substantially the same as the amount of reflected light. Note that a beam splitter may be used in place of the half mirror 173.
The beam splitter may be used to cause the ratio between the reflected light amount and the transmitted light amount to vary. The first image capturing section 171 receives the light having been transmitted through the half mirror 173. The second image capturing section 172 transmits the light reflected at the half mirror 173. When a subject is irradiated with excitation light, the first image capturing section 171 receives the fluorescent light from the subject. The second image capturing section 172 receives the reflected light from the subject. In this way, the image capturing section 112 can simultaneously capture a low image-quality motion image and a high image-quality motion image of a same subject.

The first image capturing section 171 includes an excitation light cutting filter 162 and a first image capturing element 175. The excitation light cutting filter 162 cuts the light of the wavelength band of the excitation light, from the light having been transmitted through the half mirror 173, and transmits the other wavelength bands. The first image capturing element 175 receives the light having been transmitted through the half mirror 173 from which the wavelength band of the excitation light is cut. Accordingly, when a subject is irradiated with excitation light, the first image capturing element 175 can receive fluorescent light. The second image capturing section 172 includes a color filter 152 and a second image capturing element 176. The second image capturing element 176 receives the light having been transmitted through the color filter 152 after reflected at the half mirror 173. When the irradiating section 105 irradiates a subject with white light, the second image capturing element 176 is able to capture a color image of the subject, and when the subject is irradiated with excitation light, the second image capturing element 176 can capture a background image. In addition, since the first image capturing element 175 receives fluorescent light that is weak light, the pixels thereof may have a pixel area larger than the pixels of the second image capturing element 176. When the effective pixel area is the same between the first image capturing element 175 and the second image capturing element 176, the resolution will be lower for the first image capturing element 175 than for the second image capturing element 176.

Note that the first image capturing section 171 includes a first image capturing element driver for driving the first image capturing element 175. The second image capturing section 172 includes a second image capturing element driver for driving the second image capturing element 176. The first image capturing element driver and the second image capturing element driver are controlled by an information processing apparatus such as a CPU. The information processing apparatus may be provided in the image capturing section 112, or in the image processing system 100.

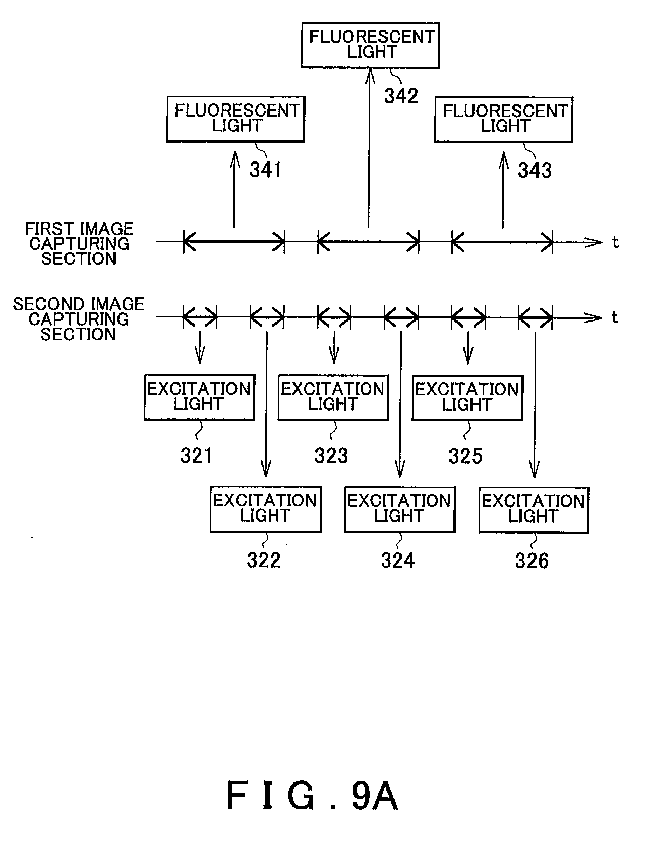

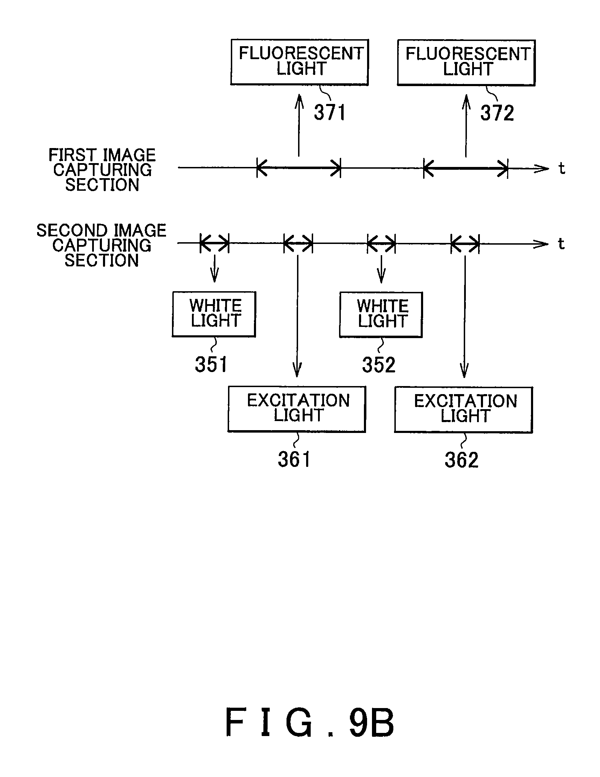

Fig. 9A and Fig. 9B show an example of a time chart of an exposure time of a first image capturing section 171 and a second image capturing section 172. Fig. 9A shows an example of a time chart of an exposure time for the first image capturing section 171 and the second image capturing section 172 when only excitation light is emitted. In this case, the rotation filter 108 is fixed to transmit, through the second filter 142, the light from the light source 107 of the irradiating section 105, thereby emitting only excitation light. The lateral axis indicates a time. A short arrow in both directions indicates an exposure time for the second image capturing section 172 that performs exposure using the reflected light of the excitation light. A long arrow in both directions indicates an exposure time for the first image capturing section 171 that performs exposure using the fluorescent light. Since the half mirror divides the light from the subject into two rays of light, and the first image capturing section 171 and the second image capturing section 172 capture the divided rays of light respectively, to be able to capture images of a same subject simultaneously. The second image capturing section 172 performs exposure using the reflected light of the excitation light, and so has an exposure time shorter than an exposure time for the first image capturing section 171 performing exposure using only the fluorescent light. Because having a shorter exposure time, the second image capturing section 172 is also able to capture an image at a frame rate higher than the frame rate of the first image capturing section 171. The second image capturing section 172 captures an excitation light image 321 by performing exposure using the reflected light of the excitation light. Then again, the second image capturing section 172 captures the excitation light image 322 by performing exposure using the reflected light of the excitation light. By performing the stated operation, the excitation light motion image is captured at a certain frame rate, in such an order as an excitation light image 321, an excitation light image 322, an excitation light image 323, an excitation light image 324, an excitation light image 325, an excitation light image 326, and so on. Simultaneously with the image capturing by the second image capturing section 172, the first image capturing section 171 captures a fluorescent light image. That is, the first image capturing section 171 captures a fluorescent light image 341 by performing exposure using fluorescent light. Then again, the first image capturing section 171 performs exposure using fluorescent light to capture a fluorescent light image 342. By performing the stated operation, the fluorescent light motion image is captured at a frame rate lower than the certain frame rate for the excitation light motion image, in such an order as a fluorescent light image 341, a fluorescent light image 342, a fluorescent light image 343, and so on. Note that the excitation light images may be used as a background image.

Fig. 9B shows an example of a time chart of an exposure time for the first image capturing section 171 and the second image capturing section 172 when irradiation of white light and excitation light is switched alternately. The lateral axis indicates a time. A short arrow in both directions indicates an exposure time for the second image capturing section 172. A long arrow in both directions indicates an exposure time for the first image capturing section 171. First, when the white light is emitted, the second image capturing section 172 captures a white light image 351 by performing exposure using the reflected light of the white light. Next, when the excitation light is emitted, the second image capturing section 172 captures an excitation light image 361 by performing exposure using the reflected light of the excitation light. During this operation, the first image capturing section 171 captures a fluorescent light image 371 by performing exposure using the fluorescent light, in an exposure time longer than an exposure time used in capturing the excitation light image 361. In addition, the exposure time in which the first image capturing section 171 performs exposure using the fluorescent light is shorter than the exposure time in which the second image capturing section 172 performs exposure using the reflected light. Next, when the white light is emitted, the second image capturing section 172 captures a white light image 352 by performing exposure using the reflected light of the white light. Next, when the excitation light is emitted, the second image capturing section 172 performs exposure using the reflected light of the excitation light, to capture an excitation light image 362. During this operation, the first image capturing section 171 captures a fluorescent light image 372 by performing exposure using the fluorescent light, in an exposure time longer than an exposure time used for capturing the excitation light image 362. The stated operation enables to capture a motion image of reflected light of white light, a motion image of reflected light of excitation light, and a motion image of fluorescent light. Note that when white light is emitted, the first image capturing section 171 may also capture an image of reflected light of the white light. In addition, the images of the reflected light of the white light captured by the first image capturing section 171 and the second image capturing section 172 simultaneously may be combined into one piece of image. The combined image will constitute a motion image of reflected light of white light. When the irradiating section 105 only emits white light, both of the first image capturing section 171 and the second image capturing section 172 will capture a motion image of reflected light of the white light.

The following explains the operation of the image processing system 100, when the image capturing section 112 is as shown in Fig. 8. Here, the irradiating section 105 is explained to only emit excitation light. By emission of only excitation light, the first image capturing section 171 can capture a fluorescent light motion image, and the second image capturing section 172 can mainly capture a motion image of reflected light of excitation light. During this operation, the second image capturing section 172 captures the reflected light of the excitation light and the fluorescent light. However, since the fluorescent light is weak, a background image can be captured. In addition, because of the weakness of the fluorescent light, the first image capturing section 171 may capture an image in an exposure time longer than the exposure time adopted by the second image capturing section 172. Also because of the weakness of the fluorescent light, the first image capturing section 171 may capture an image at a frame rate lower than the frame rate adopted by the second image capturing section 172. Furthermore because of the weakness of the fluorescent light, the pixels of the first image capturing element 175 may have a larger pixel area than the pixels of the second image capturing element 176. Moreover because of the weakness of the fluorescent light, the first image capturing section 171 may read the pixel charge of the first image capturing element 175 after adding it together.

The high image-quality motion image obtaining section 122 obtains the reflected light motion image captured by the second image capturing section 172. The low image-quality motion image obtaining section 123 captures the fluorescent light motion image captured by the first image capturing section 171. The motion calculating section 124 calculates the motion of a subject from the reflected light motion image obtained by the high image-quality motion image obtaining section 122. The image adjusting section 125 adjusts the image quality of the fluorescent light motion image obtained by the low image-quality motion image obtaining section 123, using the motion calculated by the motion calculating section 124. The image adjusting section 125 may correct the blurring in the motion image constituting images included in the fluorescent light motion image, using the motion calculated by the motion calculating section 124, when the exposure time is shorter for the reflected light motion image than for the fluorescent light motion image. When the frame rate is higher for the reflected light motion image than for the fluorescent light motion image, the image adjusting section 125 may interpolate the motion image constituting images included in the fluorescent light motion image, using the motion calculated by the motion calculating section 124. When the resolution is higher for the reflected light motion image than for the fluorescent light motion image, the image adjusting section 125 may render the fluorescent light motion image in a higher resolution, using the motion calculated by the motion calculating section 124.

The image recording section 103 records the reflected light motion image obtained by the high image-quality motion image obtaining section 122 and the fluorescent light motion image adjusted by the image adjusting section 125. The image display section 104 displays the reflected light motion image and the fluorescent light motion image recorded by the image recording section 103. The image display section 104 may display the reflected light motion image and the fluorescent light motion image simultaneously. Specifically, the first display region may display the reflected light motion image, and the second display region may display the fluorescent light motion image. In addition, the image display section 104 may display one of the reflected light motion image and the fluorescent light motion image. For example, display of the reflected light motion image and the fluorescent light motion image can be switched in response to a switching instruction from a user.

When the irradiating section 105 has switched between emitted excitation light and white light, the motion may be obtained from the motion image of the reflected light of the white light captured by the second image capturing section 172. The motion may also be obtained from the motion image of the reflected light of the excitation light captured by the second image capturing section 172. The motion may also be obtained form the motion image captured by the second image capturing section 172. In other words, the motion may be obtained from the motion images of the reflected light of the white light and of the reflected light of the excitation light captured by the second image capturing section 172.

In this way, the motion of a subject in a high image-quality motion image can be calculated from the high image-quality motion image for the purpose of adjusting the image quality of a low image-quality motion image. Therefore, the image quality of a low image-quality motion image can be adjusted with accuracy. Note that in the above-described embodiment, a reflected light motion image and a fluorescent light motion image are taken as an example of a high image-quality motion image and a low image-quality motion image. However, the high image-quality motion image and the low image-quality motion image are not limited to them. Moreover, it is possible to cause an information processing apparatus such as a CPU to function as an image processing system 100, by executing a predetermined program.

Although some aspects of the present invention have been described by way of exemplary embodiments, it should be understood that those skilled in the art might make many changes and substitutions without departing from the spirit and the scope of the present invention which is defined only by the appended claims.

The operations, the processes, the steps, or the like in the apparatus, the system, the program, and the method described in the claims, the specification, and the drawings are not necessarily performed in the described order. The operations, the processes, the steps, or the like can be performed in an arbitrary order, unless the output of the former-described processing is used in the later processing. Even when expressions such as "First," or "Next," or the like are used to explain the operational flow in the claims, the specification, or the drawings, they are intended to facilitate the understanding of the invention, and are never intended to show that the described order is mandatory.

## Claims

1. An image processing system comprising:
an image obtaining section that obtains a high image-quality motion image and a low image-quality motion image in which a same subject is captured simultaneously;
a motion calculating section that calculates a motion of the subject in the high image-quality motion image, from the high image-quality motion image; and
an image adjusting section that generates a motion image resulting from adjusting an image quality of the low image-quality motion image, based on the motion calculated by the motion calculating section.

2. The image processing system according to Claim 1, wherein
the image obtaining section obtains the low image-quality motion image generated by receiving fluorescent light from the subject irradiated with excitation light, and the high image-quality motion image generated by receiving reflected light from the subject.

3. The image processing system according to Claim 2, further comprising:
an irradiating section that sequentially irradiates the subject with the excitation light and visible light, by switching between the excitation light and the visible light; and
an image capturing section that sequentially captures images of fluorescent light from the subject irradiated with the excitation light and images of reflected light from the subject irradiated with the visible light, wherein
the image obtaining section obtains a motion image of the fluorescent light and a motion image of the reflected light that have been captured by the image capturing section.

4. The image processing system according to Claim 2, further comprising:
an irradiating section that irradiates the subject with the excitation light;
a first image capturing section that captures images of fluorescent light from the subject irradiated with the excitation light; and
a second image capturing section that captures images of reflected light from the subject irradiated with the excitation light, wherein
the image obtaining section obtains a motion image of the fluorescent light captured by the first image capturing section and a motion image of the reflected light captured by the second image capturing section.

5. The image processing system according to Claim 4, wherein
the first image capturing section captures an image in an exposure time longer than an exposure time for the second image capturing section.

6. The image processing system according to Claim 4, wherein
the first image capturing section captures an image at a frame rate shorter than a frame rate adopted by the second image capturing section.

7. The image processing system according to Claim 4, wherein
the first image capturing section captures a motion image whose resolution is lower than a resolution adopted by the second image capturing section.

8. The image processing system according to Claim 7, wherein
the first image capturing section captures the motion image whose resolution is lower than the resolution adopted by the second image capturing section, by reading accumulated charge of a plurality of pixels having been added together.

9. The image processing system according to Claim 1, wherein
the image obtaining section obtains the high image-quality motion image whose exposure time is shorter than an exposure time in which the low image-quality motion image is exposed, and
the image adjusting section generates motion image constituting images by correcting blurring in motion image constituting images included in the low image-quality motion image, based on the motion calculated by the motion calculating section.

10. The image processing system according to Claim 1, wherein
the image obtaining section obtains the high image-quality motion image whose frame rate is higher than a frame rate of the low image-quality motion image, and
the image adjusting section generates a interpolation image for interpolating motion image constituting images included in the low image-quality motion image, based on the motion calculated by the motion calculating section.

11. The image processing system according to Claim 1, wherein
the image obtaining section obtains the high image-quality motion image whose resolution is higher than a resolution of the low image-quality motion image, and
the image adjusting section renders, in a high resolution, motion image constituting images included in the low image-quality motion image, using the motion calculated by the motion calculating section.

12. The image processing system according to Claim 3, being an endoscope system including an endoscope apparatus, wherein
the irradiating section and the image capturing section are provided at a tip of an insertion section of the endoscope apparatus.

13. The image processing system according to Claim 4, being an endoscope system including an endoscope apparatus, wherein
the irradiating section, the first image capturing section, and the second image capturing section are provided at a tip of an insertion section of the endoscope apparatus.

14. An image processing method for processing an image by means of a computer, comprising:
obtaining a high image-quality motion image and a low image-quality motion image in which a same subject is captured simultaneously;
calculating a motion of the subject in the high image-quality motion image, from the high image-quality motion image; and
adjusting an image quality of the low image-quality motion image, using the calculated motion.

15. A computer readable medium for storing a program, the program causing a computer to function as:
an image obtaining section that obtains a high image-quality motion image and a low image-quality motion image in which a same subject is captured simultaneously;
a motion calculating section that calculates a motion of the subject in the high image-quality motion image, from the high image-quality motion image; and
an image adjusting section that adjusts an image quality of the low image-quality motion image, using the motion calculated by the motion calculating section.
